# EUROPEAN PATENT APPLICATION

(11) **EP 4 714 336 A1**
(43) Date of publication of application: **25.03.2026**
(21) Application number: 25152010.2
(22) Date of filing: 15.01.2025
(51) Int. Cl.: A61B 5/00, A43B 17/00, A61B 5/026, A61B 5/103, A61B 5/11

(54) **SENSING DEVICE FOR DETECTING FOOT DATA**

(30) Priority: 19.09.2024 KR 20240126927
(71) Applicant: Decentralized Biotechnology Intelligence Co., Ltd., Taipei City, Da'an Dist. 106021 (TW)
(72) Inventor: CHOU, Yao-Sheng, 106021 Taipei (TW); JIANG, Lin-Yi, 106021 Taipei (TW); CHI, Hsing-Yu, 106021 Taipei (TW); CHOU, Yen-Han, 106021 Taipei (TW)
(74) Representative: Schmidt, Steffen J.

(57) **Abstract**

A foot sensor and analysis device, which includes a pressure sensing layer arranged inside the insole and a sensing module installed inside the insole. The sensing module is electrically coupled with the pressure sensing layer for receiving and processing detected electronic signals, where sensing module includes an inductance coil to perform wireless charging to the battery. The pressure sensing layer and the sensing module are integrally formed inside the insole.

## Description

### TECHNICAL FIELD

The present invention relates to a sensor, and more particularly, to a foot sensor and analysis device.

### BACKGROUND

Recently, due to the development of electronic technology, in addition to improvements of accuracy of precision electronic sensors, these sensors have been developed toward to lightweight and portable designs that provide break through beyond the limitations of conventional use, enabling that wearable technologies designed for recording daily physical activities or for professional sports have developed rapidly. However, recently developed functionalities are mainly on simple calculations of step count and stride frequency. In fact, the sensing elements of a wearable device can capture a lot of data during its use. How to further analyze and use these sensed data is the focus of developments in future wearable technology.

Miniaturized electronic sensing components can allow sensors to be installed in shoes, allow experiments to be performed beyond the indoor environment. Also, measurements of the plantar pressure in shoes can also bring additional advantages to motion sensing. Taking running as an example, when the lower limbs are in contact with the ground, despite that instruments such as force plates can accurately measure the external force on the human body, only the plantar pressure can simultaneously provide the time and space parameters of the external force within the plantar area. For example, different forces acting on specific locations of the sole of foot may have different meanings, which may cause the change the probability of sports injuries or falls. The information obtained depends on plantar pressure, which cannot be obtained from other measurement tools. This uniqueness makes the importance of plantar pressure for clinical and sports-related detection cannot be ignored.

The data of plantar pressure distribution can reveal the gait pattern of human body. The measurement of the plantar pressure distribution has great reference value in the fields of biomechanics, rehabilitation medicine, sports training, shoe making and so on. At present, both the clinical pressure test plate and test bench have space limitations and they are both not wearable.

The existing sensors for testing plantar pressure, because its sensing unit is in contact with people's feet, it is easy to wear and tear due to frequent contact with the soles of the feet, which is not conducive to long-term wearing and testing.

Therefore, developing a foot sensor and analysis device, enabling that the sensors can be embedded inside the insole, and the sensor and the insole can be integrally formed during production, can further solve the above deficiencies.

### SUMMARY OF THE INVENTION

The purpose of the present invention is to disclose a foot sensing device for detecting the foot data or information. According to one aspect of the present invention, the foot data sensing device includes a pressure sensing layer arranged inside the insole and a sensing module installed inside the insole. The sensing module is electrically coupled with the pressure sensing layer for receiving and processing detected electronic signals, where sensing module includes an inductance coil to wireless charge the battery. In an embodiment, the pressure sensing layer and the sensing module are integrally formed inside the insole.

In an embodiment, the foot data sensing device further comprises an infrared sensing layer disposed inside the insole and electrically connected to the sensing module to transmit electronic signals detected by the infrared sensing layer, wherein the infrared sensing layer is integrally formed inside said insole.

In an embodiment, the pressure sensing layer includes a plurality of pressure sensors arranged with different density distribution, which are arranged on a forefoot area, a lateral arch area and a heel area in said insole.

In an embodiment, the pressure sensing layer comprises a plurality of resistive pressure sensors. In one case, the pressure sensing layer and the infrared sensing layer is flexible. In an embodiment, the pressure sensing layer includes a plurality of capacitive sensors with different density distribution, which are arranged on a forefoot area, a lateral arch area and a heel area in said insole.

In an embodiment, the sensing module provides program or algorithm to control collection and storage of data. The insole further comprises an accelerometer used to detect direction changes, GPS data, acceleration output data, angular orientation related data, and angular orientation changes during the user's walking, for detecting information including speed/distance, and to correlate with sensed pressure data for cross reference and correction.

According to another aspect of the present invention, the sensing module includes a processing unit to collect and analyze the electrical signals sensed by the pressure sensing layer and the infrared sensing layer to convert the electrical signals to a corresponding foot pressure distribution and a blood circulation information. A memory is coupled to the processing unit to store the corresponding foot pressure distribution and the blood circulation information. A wireless data transceiver is coupled to the processing unit to transmit the corresponding foot pressure distribution and the blood circulation information to an external electrical device.

In one embodiment, the present invention includes a power supply unit to provide power to a pressure sensing layer, the infrared sensing layer, the processing unit, the memory and the wireless data transceiver.

In one embodiment, the pressure sensing layer includes a plurality of pressure sensors arranged with different density distribution, which are arranged on a forefoot area, a lateral arch area and a heel area in said insole. The sensing module includes program or algorithm to control data collection and data storage.

In one embodiment, the sensing module is configured to communicate with an external computing device, for example, a mobile device or other electronic device. In one embodiment, the wireless data transceiver includes a Bluetooth, a wireless fidelity, 5G, 6G communication device or the combination thereof. The external computing device is coupled to the edge computing device which is coupled to the cloud computing device. Edge computing is a type of computing network architecture, where computation is located as close to the source of data as possible to reduce latency and bandwidth use. Edge computing brings enterprise applications closer to data sources such as IoT devices or local edge servers. Edge computing and mobile edge computing on 5G, 6G networks enables faster and more comprehensive data analysis.

### BRIEF DESCRIPTION OF THE DRAWINGS

FIG. 1A shows a top view of an insole with integrated formed sandwich sensors in accordance with one embodiment of the invention.
FIG. 1B shows a side view of an insole with integrated formed sandwich sensors in accordance with one embodiment of the invention.
FIG. 1C shows a distribution diagram of pressure sensors in an insole with integrated formed sandwich sensors in accordance with one embodiment of the invention.
FIG. 1D shows a cross-sectional view of an insole with integrated formed sandwich sensors in accordance with one embodiment of the invention.
FIG. 2 shows a functional block diagram of an insole sensing system in accordance with one embodiment of the invention.
FIG. 3 shows a functional block diagram of a left and a right insole sensing system communicated with an external computing device in accordance with one embodiment of the invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

Plantar pressure is referred to the force per unit area of the human body when the sole of the foot touches the ground during various forms of motion. The plantar pressure detection system includes multiple pressure sensing elements. The plantar pressure parameters can be obtained by the collected and calculated pressure values from each pressure sensing element during the measurement process.

At present, the pressure sensing elements used to detect the plantar pressure can be mainly divided into two types, i.e., capacitive and resistive types of pressure sensing element. The capacitive pressure sensing element uses a diaphragm to separate two conductive plates. When the diaphragm on the sensing element is deformed by pressure, the gap between the diaphragm and the two conductive plates changes, resulting in a change in capacitance, and the magnitude of the pressure can be estimated by measuring the change in capacitance.

The resistive pressure sensing element includes a conductive polymer, and the conductive polymer changes resistance as the pressure changes. The conductive particles of the conductive polymer can be brought into contact by applying force onto, the current through the sensing element is therefore enhanced and the pressure can be calculated.

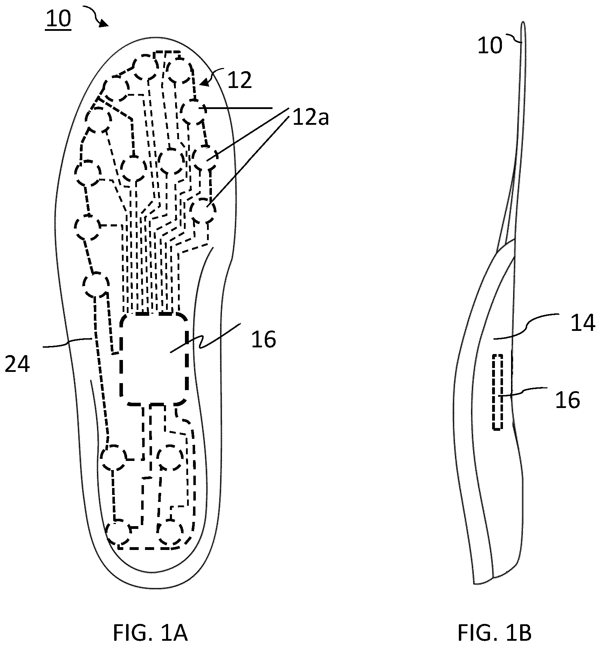

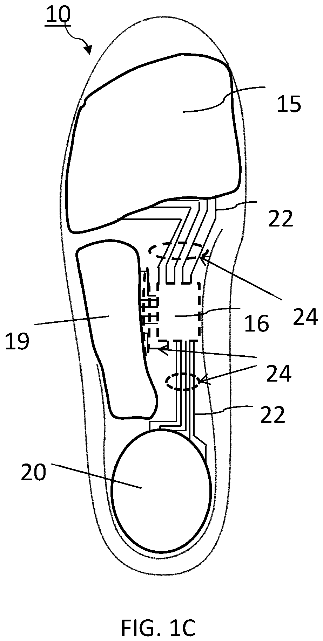

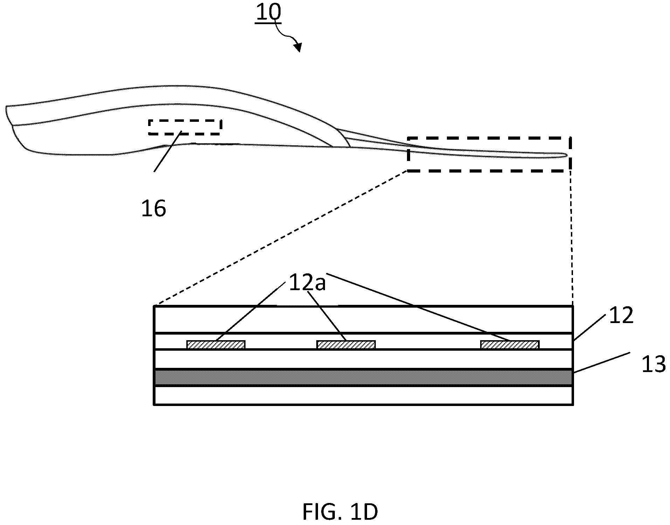

FIG. 1 shows an insole 10 with integrated formed sandwich sensors according to an embodiment of the present invention. FIG. 1A shows a top view of the insole 10. The pressure sensing layer 12 is embedded inside the insole, and illustrates by dashed lines. The pressure sensing layer 12 includes a plurality of pressure sensors 12a to form an array configuration, individual pressure sensors 12a acts as a sensing point to sense the pressure changes and position distribution when the foot pressure changes. The pressure sensor 12a is electrically connected to the sensing module 16 through a wire 24.

In a preferred embodiment, the pressure sensor 12a shown in FIG. 1A is a resistive pressure sensing element, which includes a conductive polymer, and the conductive polymer changes resistance as the pressure changes. The conductive particles of the conductive polymer can be brought into contact by applying force onto, the current through the sensing element is therefore enhanced and the pressure can be calculated.

In another preferred embodiment, the pressure sensor 12a shown in FIG. 1A is a capacitive pressure sensor, which includes a diaphragm to separate two conductive plates. When the diaphragm on the sensing element is deformed by pressure, the gap between the diaphragm and the two conductive plates changes, resulting in a change in capacitance, and the magnitude of the pressure can be estimated by measuring the change in capacitance.

FIG. 1B shows a side view of the insole 10. The pressure sensing layer 12 of the insole 10 is integrated formed with sandwich sensors. The insole 10 may include a thermoplastic polyester elastomer (TPEE) layer, and may also include an arch support (cushion) 14 integrated therein. A sensing module 16 is embedded in the arch support 14 of the insole, used to collect, calculate, process and transmit the electronic signals of a plurality of pressure sensors, which can avoid or minimize the contact and stimulation to the wearer's foot.

In a preferred embodiment, the sensing module 16 is integrated and packaged with a flexible substrate disposed in the insole 10 in an integrated molded manner.

As shown in Fig. 1C, the pressure sensors (not shown) in the whole insole can be configured at the positions, such as in the toe area 15, the lateral arch area 19 and the heel area 20, with different density distributions. Each of the pressure sensors is electrically connected to the sensing module 16 by wirings 22. In one embodiment, the pressure sensors with different numbers and corresponding wiring 24 form a flexible pressure sensing device, which is arranged in different parts of the insole to sense the foot pressure distribution of different areas (for example, the positions near the toe area 15, the lateral arch area 19 and the heel area 20) of the user's foot. In other words, the sensing densities of the above three areas are different, in which the density is in order from small to large, i.e., forefoot area (near toe area) 15, lateral arch area 19 and heel area 20. Among them, the sensing module 16, including a wireless data transceiver 32 and other electronic components, is embedded in the arch support 14 of the arch part of the insole 10. In one embodiment, the sensing module 16 may be an electronic sensing module integrated by a printed circuit board, which has a connection terminal electrically connected with the plurality of pressure sensing devices. The size of the sensing module 16 can be miniaturized, and its thickness is about 3 ~ 4.5 mm (millimeter).

FIG. 1D illustrates a cross-sectional view of the insole 10, and the figure below it is an enlarged partial sectional view of the insole 10 located in the dotted box area. In the figure, individual pressure sensors 12a are arranged in a flexible substrate with corresponding wirings connected, so that individual pressure sensors 12a can transmit the sensed signals to the sensing module 16 through the wirings. In one embodiment, the pressure sensing layer 12 includes a flexible pressure sensor 12a and corresponding wirings.

In addition, the insole 10 can also integrate with an infrared sensor to detect the blood circulation of the user's foot. Based on the high penetration of infrared, the infrared sensor can detect the blood circulation of the foot without clinging to the human skin. FIG. 1D shows that an infrared sensing layer 13 includes a flexible infrared sensor and wiring, which is arranged inside the insole 10 to receive the infrared emitted from the human foot, and this configuration can avoid mutual interference with the pressure sensor. In one embodiment, the infrared sensing layer 13 can be electrically connected with the connection terminal of the sensing module 16 through the flexible wiring. Through the high penetration of the above infrared, the artery on the outside of the instep responsible for delivering blood to the foot is measured, so as to judge whether the instep pulse may be abnormal.

In an embodiment, the pressure sensing layer 12, infrared sensing layer 13 and sensing module 16 can be made flexible, so that the pressure sensing layer 12, the infrared sensing layer 13 and the sensing module 16 can be integrally embedded inside the insole 10 when the insole 10 is injection molded. Among them, the insole 10 adopts the wireless charging mode, the overall insole can be completely free of exposed holes, and the insole 10 can be cleaned in a washing machine.

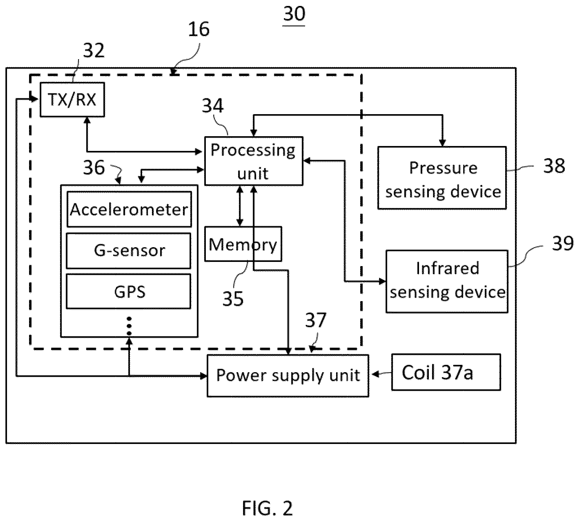

FIG. 2 shows a functional block diagram of an insole sensing system 30, which includes a sensing module 16 to transmit data by a wireless data transceiver (TX/RX) 32. The wireless data transceiver (TX/RX) 32 includes a Bluetooth, a wireless fidelity, 5G, 6G communication device or the combination thereof. As depicted in FIG. 2, the wireless data transceiver (TX/RX) 32 is integrated into the sensing module 16, those skilled in the art can understand that the wireless data transceiver (TX/RX) 32 can also be a separate component for the purpose of data transmission/receiving. In the example of FIG. 2, the sensing module 16 includes a wireless data transceiver (TX/RX) 32 for transmitting data to and/or receiving data from one or more remote systems. In one embodiment, the wireless data transceiver (TX/RX) 32 includes a Bluetooth, WiFi (Wireless Fidelity), 5G, 6G communication device or similar wireless data transceiver. The sensing module 16 can be electrically connected with a plurality of pressure sensors (pressure sensing device 38) and an infrared sensing device 39 arranged in the insole via a connection terminal. The sensing module 16 also includes a processing unit 34 (such as, one or more microprocessors), a memory 35, additional sensors 36 (including accelerometer, gyroscope (G-sensor), global positioning system (GPS) sensor, etc.) 36, and a power supply unit 37. The power supply module 37 can provide electrical power to the pressure sensing device, infrared device 39 and/or other components of the sensing system (for example, processing unit 34, memory 34, additional sensors 36). In one preferred embodiment, the power supply module 37 includes a rechargeable solid-state battery, an induction coil 37a (for coupling with an external wireless charging system to charge the battery 37 wirelessly) and a USB charging interface. It should be realized that the sensing module 16 can provide computer programs/algorithms to control the collection and storage of data (for example, user's foot pressure distribution data or pressure data interacting with the ground, user's foot blood cycle state, etc.), and these programs/algorithms can be stored and/or executed.

The TX/RX device 32 can connect to one or more sensors, and transmit or provide the detection data or information related to various different parameters created by the additional sensors 36. These data or information include physiological data related to the user, speed data/distance information of pedometer type. The accelerator is used to detect change of directions during walking detected by accelerometer, GPS data, acceleration output/data, angular orientation related data and change of angular orientation (sensing by G-sensor), and these data can be stored in the memory or transmitted to a remote computing device or server via the TX/RX device 32.

In the embodiment of Fig. 2, the sensing module 16 may include a startup system (not shown). That is, the system or a part thereof can be coupled to the sensing module 16 or connected to the insole or separated from other parts of the sensing module 16. The startup system may be used to selectively start the sensing module 16. In one embodiment, the sensing module 16 may be operated through a specified mode to enable the sensing module to be started or closed, such as a pressure threshold applied to one or more sensors. In other embodiments, the sensing module 16 may be turned on or off through a button. In any of these embodiments, the sensing module 16 may include a sleep mode that may put the system into the sleep mode after a period of inactive state. In one embodiment, for example, the G-sensor does not detect activity within a preset time, and the sensing module 16 may enter sleep mode to save power.

The sensing module 16 may also be configured to communicate with an external device, which may be an external computing device, a computing system, a mobile device (smart phone, tablet, etc.), or other electronic devices.

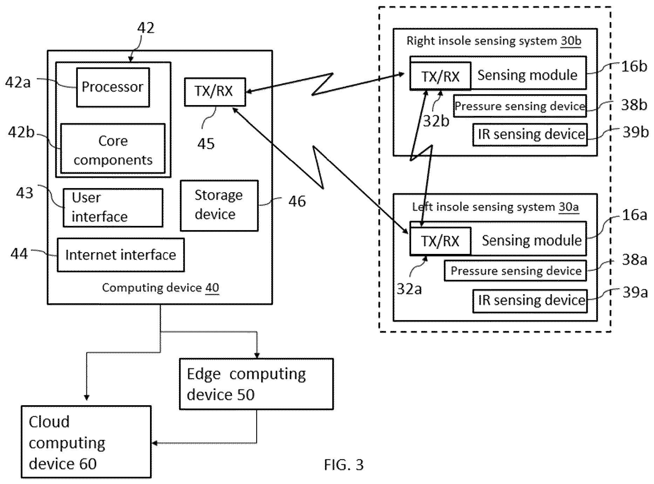

Fig. 3 shows a system block diagram of communication between the left and right insole sensing systems (30a, 30b) and an external computing device 40. The left and right insole sensing systems (30a, 30b) each includes a sensing module (16a, 16b) embedded in the arch of the insole, which is electrically connected with a pressure sensing devices (38a, 38b) and an infrared sensing device (39a, 39b) to receive and analyze a pressure distribution of the user's foot and a blood circulation data of foot, and transmit the above data to a remote computing device or server through a TX/RX device (32a, 32b) located in the sensing module. The sensing modules (16a, 16b) each include a processing unit (such as, one or more microprocessors), a memory, additional sensors, and a power supply unit (referring to Fig. 2).

The external computing device 40 is any electronic device that can transmit data, process data, and/or store data. In one embodiment, the computing device 40 is a portable computing device and/or a fixed computing device. The portable computing device may be a social network device, a game device, a mobile phone, a smart phone, a personal digital assistant, a digital audio/video player, a notebook computer, a tablet computer, a video game controller, and/or any other portable device containing a computing core. The fixed computing device may be a personal computer (PC), a computer server, a television, a printer, a fax machine, a home entertainment device, a video game console, and/or any type of home or office computing device containing a computing core.

The external computing device 40 includes a computing core 42, a user interface 43, an Internet interface 44, a wireless communication transceiver 45, and a storage device 46. The user interface 43 includes one or more input devices (such as, keyboard, touch screen, voice input device, etc.), one or more audio output devices (such as, speaker, headphone jack, etc.), and/or one or more video output devices (such as, video graphics display, touch screen, etc.). The Internet interface 44 includes one or more networking devices (such as, wireless local area network (WLAN) devices, wired LAN devices, wireless wide area network (WWAN) devices, etc.). The storage device 46 includes a flash memory device, one or more hard disk drives, one or more solid-state (SS) storage devices, and/or a cloud memory.

The computing core 42 includes a processor 42a and other computing core components 42b. Other computing core components 42b include a video graphics processing unit, a memory controller, a main memory (such as RAM), one or more input/output (I/O) device interface modules, input/output (I/O) interfaces, input/output (I/O) controllers, peripheral device interfaces, one or more USB interface modules, one or more network interface modules, one or more memory interface modules and/or one or more peripheral device interface modules.

The wireless communication transceiver 45 of the external computing device 40 and the wireless data transceiver (32a, 32b) of the insole sensing system 30 have similar transceiver types (such as, Bluetooth, WiFi, 5G, 6G communication device etc.). The wireless data transceiver (32a, 32b) communicates directly with the wireless communication transceiver 45 to share the collected data and/or receive instructions from the external computing device 40 through the respective insole sensing system 30. An alternative example, the wireless data transceiver (32a, 32b) communicates with one of them to collect data. The wireless data transceiver 32a transmits the collective data to the wireless communication transceiver 45 of the external computing device 40.

The external computing device 40 is coupled to the edge computing device 50 which is coupled to the cloud computing device 60. Edge computing is a type of computing network architecture, where computation is moved as close to the source of data as possible, in order to reduce latency and bandwidth use. The aim is to reduce the amount of computing required to be performed in a centralized, remote location (i.e. the "cloud") far away from the source of the data, thus minimizing the amount of long-distance communication between a client and server. Edge computing brings enterprise applications closer to data sources such as IoT devices or local edge servers. This proximity to data can deliver strong business benefits, including improved response times and better bandwidth availability. Edge computing and mobile edge computing on 5G networks enables faster and more comprehensive data analysis. Thus, in one preferred embodiment, the insole data is performed by the edge computing device 50 and/or the external computing device 40 by using the 5G OR 6 G network. The task that cannot be processed by the edge computing device 50 will be sent to the cloud computing device 60.

The edge computing architecture can be divided into: "device layer" for data collection, "edge layer" for real-time data processing, and "cloud layer" responsible for secure storage and in-depth analysis. In the embodiment, each sensing device collects foot data through built-in sensors. For example, the pressuring sensors collect dynamic data of the feet. The "edge layer" is located closest to where data is generated, and its distribution is wider than traditional cloud servers. Able to perform real-time processing and analysis of data, significantly reducing latency. For example, the edge computing device will analysis user's foot pressure distribution data, user's foot blood cycle state, user physiological data, speed/distance information, pedometer information, the location of the position, gait, cadence, the center of pressure (COP) and movement information.

If the data requires deeper analysis, the information will be uploaded to the "cloud layer" for further analysis. Although edge computing solves the bottleneck and delay problems of cloud computing, when the "edge layer" determines that some data requires more detailed analysis, it will send the data to the "cloud layer" for deeper computing and storage. For example, the health state of the user.

The external computing device 40 processes data to produce various results. For example, the external computing device 40 processes the data from the sensing system 16 in combination with the circuit of algorithm, which can analyze any data related to foot pressure during movement, such as the pressure distribution on the wearer's left and right feet, the ratio of weight to the left and right feet, gait, cadence, and the center of pressure (COP) during body dynamics.

Foot pressure distribution plays a critical role in a movement of human body. A posture of human body and changes in the bone are affected by foot shape and walking (running) posture, which also affects the performance and limit in sports. The invention proposes an insole with integrated formed sandwich sensors, which can obtain the parameter data of foot pressure distribution of many users for time and space through the insole arranged in the shoe, and upload the data to external computing devices (e.g. smart phone, personal computer, computer servers, etc.) to calculate, analyze and store the data in the cloud system as relevant database of big data.

In addition, the insole with integrated formed sandwich sensors can also integrate an infrared detection device to synchronously provide the user's blood circulation information. Breaking through the limitation that only medical institutions or sports research institutions can obtain data analysis in the past, the invention can facilitate more sports and more users obtaining exclusive movement or motion analysis. Synchronously, it also enables the establishment and use of data platforms in various professional fields, so that different professionals (such as sports, health care, shoemaking, etc.) can establish their linkage relationship with foot pressure performance.

In one embodiment, the above-mentioned data is transmitted wirelessly, while combined with the APP, it can be displayed in real time, so that the above-mentioned data can be visualized.

The above-mentioned data collected by the insole with integrated formed sandwich sensors can be applied in more diverse sports and lets more users to obtain exclusive personal movement or sports analysis. Simultaneously, it also opens up the establishment and use of data platforms in various professional areas, so that different professional sports can establish a linkage between sport and foot pressure performance, and further develop various algorithms and apps, thereby unlocking the mysteries of human movement and posture.

In addition, the sensing insole proposed by the present invention adopts a wireless charging mode, the entire insole can be completely free of exposed holes, and the insole can be washed in a washing machine; moreover, the sensing module and the insole can be in integrally formed during injection molding.

While various embodiments of the present invention have been described above, it should be understood that they have been presented by a way of example and not limitation. Numerous modifications and variations within the scope of the invention are possible. The present invention should only be defined in accordance with the following claims and their equivalents.

## Claims

1. A foot data sensing device, comprising:
pressure sensors (38);
a sensing module (16) electrically coupled with said pressure sensors (38) to receive detected signals;
an inductance coil (37a) coupled with a battery (37) for wirelessly charging to provide power to said sensing module (16); and
wherein said sensing module (16) includes a wireless transceiver (32) to transmit detected signals to an external computing device (40).

2. The device of claim 1, wherein said wireless transceiver (32) includes a Bluetooth, WiFi (Wireless Fidelity), 5G, 6G communication device.

3. The device of claim 1, wherein said external computing device (40) is coupled to an edge computing device (50) or a cloud computing device (60).

4. The device of claim 3, wherein said edge computing device (50) is coupled to a cloud computing device (60).

5. The device of claim 1, wherein said pressure sensors (38) and said sensing module (16) are formed in an insole (30).

6. The device of claim 5, further comprising an infrared sensing device (39) disposed inside said insole (30) and electrically connected to said sensing module (16).

7. The device of claim 5, wherein said pressure sensors include (38) a pressure sensing layer (12) having a plurality of pressure sensors (12a) arranged with different density distribution.

8. The device of claim 7, wherein said pressure sensing layer (12) is flexible.

9. The device of claim 7, wherein said pressure sensing layer (12) includes a plurality of resistive pressure sensors.

10. The device of claim 7, wherein said pressure sensing layer (12) includes a plurality of capacitive sensors.

11. The device of claim 1, wherein said sensing module (38) is used to collect data.

12. The device of claim 1, further comprising additional sensors (36) including an accelerometer.

13. The device of claim 1, further comprising additional sensors (36) including a G sensor.

14. The device of claim 1, further comprising additional sensors (36) including a GPS.

15. The device of claim 1, wherein said pressure sensors (12a) are arranged on a forefoot area (15), a lateral arch area (19) and a heel area (20).
